# EUROPEAN PATENT SPECIFICATION

(11) **EP 1 130 102 B1**
(45) Date of publication and mention of the grant of the patent: **20.10.2004**
(21) Application number: 00870028.8
(22) Date of filing: 21.02.2000
(51) Int. Cl.: C12N 15/56, C12N 9/42, A23L 1/105, A21D 8/04, A23L 2/84, C02F 3/34, D21C 5/00

(54) **Enzyme with xylanase activity**
Enzym mit Xylanaseaktivität
Enzyme possédant une activité xylanasique

(43) Date of publication of application: 05.09.2001
(73) Proprietor: PURATOS N.V., 1702 Groot-Bijgaarden (BE)
(72) Inventor: Jonniaux, Jean-Luc, 3300 Tienen (BE); Dauvrin, Thierry, 4218 Couthuin (BE)
(74) Representative: Van Malderen, Joelle

(56) References cited:
- WO-A-94/14965
- Nucleotide Sequence Database EMBL Emfun ID: AB035540; AC: AB035540 18 December 1999 Penicillium sp.40 xynA gene coding for xylanase A 73% identity with SEQ ID NO 8 in 649bp overlap from nt.1034 to nt.1666. The encoded polypeptide is 72% identical to SEQ ID NO 11 over its entire length XP002143462

## Description

### Field of the invention

The present invention relates to an enzyme with xylanase activity identified by its amino acid and nucleotide sequences and variants thereof.

The present invention relates also to their uses in the agrofood and in the pulp and paper industries.

### Background of the invention

Xylans are heteropolysaccharides which form the major part of the hemicellulose present in the plant biomass.

The backbone of these polysaccharides is a chain of β 1-4 linked xylopyranosyl residues. Many different side groups could bind to these residues like acetyl, arabinosyl and glucuronosyl residues. Phenolic compounds such as ferulic or hydroxycinnamic acids are also involved through ester binding in the cross linking of the xylan chains or in the linkage between xylan and lignin chains for example.

Endoxylanases hydrolyse specifically the backbone of the hemicellulose. In some cases, the side groups may mask the main chain by steric hindrance. Different xylanase activities already described are characterised by their specificity towards their substrate and the length of the oligomers produced.

These differences between the xylanases concerning their properties seem to be partly related to their respective amino acid sequences. Endoxylanases have been classified into two families (F or 10 and G or 11) according to their sequence similarities (Henrissat & Bairoch, Biochem. J., Vol. 293, p. 781 (1993)). The F family of xylanases are larger, more complex as compared to the G family of xylanases. Moreover the F family xylanases produce small oligosaccharides, while the G family xylanases show a higher affinity for unsubstituted xylan.

Xylanases are used in various industrial areas such as the pulp, paper, feed and bakery industries. Other applications lie in the juice and beer sectors. Xylanases could also be used in the wheat separation process. The observed technological effects are, among others, improved bleachability of the pulp, decreased viscosity of the feed or changes in dough characteristics.

Many different microbial genera have been described to produce one or several xylanases. These microbial genera comprise bacteria as well as eukaryotic organisms like yeast or fungi. Kimura et al. (1999) have disclosed the complete coding sequence of the acidophilic xylanase A from Penicillium sp. 40 (deposited with EMBL under accession number AB0 355 40).

### Detailed description of the invention

A first aspect of the present invention is related to an isolated and purified (from possible contaminants) xylanase amino acid sequence presenting more than 80, preferably more than 85%, more preferably more than 90% sequence identity with the amino acid sequence SEQ ID NO 11.

Advantageously, the isolated and purified xylanase amino acid sequence according to the invention has a molecular weight comprised between 22 and 26 kD, preferably a molecular weight about 24 kD.

Said xylanase amino acid sequence or peptide is extra-cellular or intra-cellular expressed and/or secreted by the recombinant host cell according to the invention.

According to another preferred embodiment of the present invention, the isolated and purified xylanase amino acid sequence has the amino acid sequence of SEQ ID NO 11 or a smaller portion of said amino acid sequence (of more than 30 or 50 amino-acids, preferably more than 100 amino-acids), which has at least more than 80% of the xylanase activity of the complete amino acid sequence SEQ ID NO 11, preferably more than 95% of the xylanase activity or the complete xylanase activity of the complete amino acid sequence SEQ ID NO 11 (see also example 1). In other words, the isolated and purified xylanase amino acid sequence according to the invention may be deleted partially while maintaining its enzymatic activity, which may be measured by methods well known by the person skilled in the art.

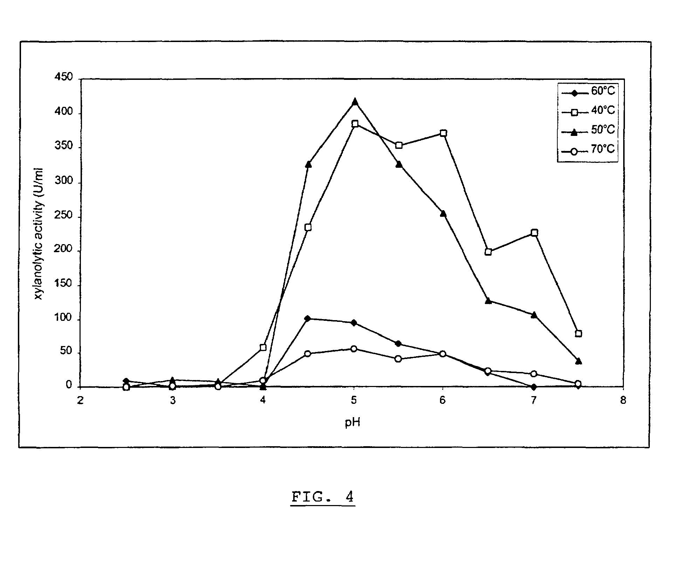

The purified xylanase enzyme according to the invention is also characterised by an optimum pH around pH 5.0 and temperature profile having its maximum activity at about 50 °C. More generally, the maximum activity of the enzyme is comprised between pH 4.5 and 7.0, at a temperature comprised between 35 and 55 °C (see Fig. 4).

The present invention is also related to an isolated and purified nucleotidic sequence from a micro-organism origin, encoding a xylanase. Preferably, said micro-organism is selected from the group consisting of bacteria or fungi (including yeast), preferably the *Penicillium* species fungi, more specifically the *Penicillium griseofulvum*.

According to a preferred embodiment of the present invention, said micro-organism is *Penicillium griseofulvum* having the deposit number MUCL-41920.

According to the invention, said nucleotide sequence presents more than 80%, more preferably more than 90% sequence identity with the sequence SEQ ID NO 8 described hereafter.

According to a preferred embodiment of the present invention, said isolated and purified nucleotide sequence corresponds to the nucleotide sequence SEQ ID NO 8 or a portion thereof encoding a peptide having a xylanase activity.

It is meant by "a portion of the nucleotide sequence SEQ ID NO 8", a fragment of said sequence SEQ ID NO 8 having more than 90 nucleotides, preferably more than 100 nucleotides or more than 120 nucleotides, of said nucleotide sequence and encoding a protein characterised by a xylanase enzymatic activity similar to the xylanase activity of the complete amino-acid sequence SEQ ID NO 11. Preferably, said portion has a xylanase enzymatic activity of more than 80% of the initial xylanase enzymatic activity of the complete enzyme defined by its amino acid sequence SEQ ID NO 11, preferably has a xylanase enzymatic activity corresponding the one of the amino acid sequence SEQ ID NO 11.

Another aspect of the present invention is related to a recombinant nucleotide sequence comprising, operably linked to the nucleotide sequence according to the invention and above-described, one or more adjacent regulatory sequence(s), preferably originating from homologous micro-organisms.

However, said adjacent regulatory sequences may also be originating from heterologous micro-organisms.

These adjacent regulatory sequences are specific sequences such as promoters, secretion signal sequences and terminators.

Another aspect of the present invention is related to the vector comprising the nucleotide sequence(s) according to the invention, possibly operably linked to one or more adjacent regulatory sequence(s) originating from homologous or from heterologous micro-organisms.

It is meant by "a vector", any biochemical construct which may be used for the introduction of a nucleotide sequence (by transduction, transfection, transformation, infection, conjugation, etc.) into a cell. Advantageously, the vector according to the invention is selected from the group consisting of plasmids, viruses, phagemids, chromosomes, transposons, liposomes, cationic vesicles or a mixture thereof. Said vector may comprise already one or more of the above-described adjacent regulatory sequence(s) (able to allow its expression and its transcription into a corresponding peptide by said micro-organism). Preferably, said vector is a plasmid incorporated in *E. Coli* and having the deposit number LMBP-3987.

The present invention is also related to the host cell, preferably a recombinant host cell, "transformed" by the nucleotide sequence or the vector according to the invention above-described.

It is meant by "a host cell "transformed" by the nucleotide sequence or the vector according to the invention", a cell having incorporated said nucleotide sequence or said vector and which does not comprise naturally (originally) said nucleotide sequence. The transformed host cell may be also a cell having incorporated said vector or said nucleotide sequence by genetic transformation, preferably by homologous recombination or other method (recombinant microorganism).

A "host cell" may be also the original cell comprising the nucleotide sequence encoding the enzyme according to the invention and genetically modified (recombinant host cell) to overexpress or express more efficiently said enzyme (better pH profile, higher extra cellular expression,...).

Preferably, said host cell is also capable of overexpressing (higher expression than the expression observed in the initial microorganism) said nucleotide sequence or said vector and allows advantageously a high production of an amino acid sequence encoded by said nucleotide sequence or by said vector. The isolated and purified nucleotide sequence according to the invention may be either integrated into the genome of the selected host cell or present on an episomal vector in said host cell.

Advantageously, the recombinant host cell according to the invention is selected from the group consisting of the microbial world, preferably bacteria or fungi, including yeast.

Preferably, said recombinant host cell is modified to obtain an expression of the xylanase enzyme at high level obtained by the use of adjacent regulatory sequences being capable of directing the overexpression of the nucleotide sequence according to the invention in the recombinant host cell or by increasing the number of nucleotide copies of the sequences according to the invention.

The following description describes also the conditions (culture media, temperature and pH conditions, etc.) for the culture of the host selected for the expression of the xylanase according to the invention. For this purpose, the original production species and/or a suitable host cell transformed with a DNA construct designed to express the said enzyme are present in a suitable growth medium.

According to the present invention, said protein with xylanolytic activity may be isolated from the medium and/or purified. The culture, isolation and purification conditions are derived from conventional methods well-known to persons skilled in the art.

The xylanase enzyme according to the invention may be used in different kinds of industries.

The enzyme with xylanolytic activity of the present invention, purified or not purified, is particularly suited as a bread-improving agent. Bread-improving agents are products which could improve or increase texture, flavour, anti-staling effect, softness, crumb softness upon storage, freshness and machinability, volume of a dough and/or of a final baked product. Preferably, said enzyme with xylanolytic activity increases the specific volume of the final baked product.

"Baked product" intends to include any product prepared from dough, in particular a bread product. Dough is obtained from any type of flour or meal (e.g. based on rye, barley, oat or maize), preferably prepared with wheat or with mixes including wheat.

A further aspect of the present invention relates to the additive effect of said enzyme having xylanolytic activity with other enzymes, in particular with an alpha-amylase, preferably an alpha-amylase from *Aspergillus oryzae.* Said enzyme with xylanolytic activity may be used in combination with other bread-improving agents like enzymes, emulsifiers, oxidants, milk powder, fats, sugars, amino acids, salts, proteins (gluten, cellulose binding site) well known to the person skilled in the art.

According to the present invention, the enzyme with xylanolytic activity, purified or not, shows hydrolytic activities in presence of plant cell wall components. Particularly, said enzyme degrades the wheat cell wall components. Particularly, the degradation activities lead to a decrease of the flour viscosity in the presence of water. Said enzyme may thus advantageously be used in the separation of components of plant cell materials such as cereal components. Particularly, said enzyme may be used to improve the separation of the wheat into gluten and starch by the so-called batter process.

According to the present invention, said enzyme may be used to improve the filtrability and/or decrease the viscosity of glucose syrups obtained from impure cereal starch by subjecting the impure starch first to the action of an alpha-amylase, then to the action of said xylanase. It may also be used in beer brewing when cereal has to be degraded to improve the filtrability of the wort or to reuse the residuals from beer production for e.g. animal feed. Said enzyme may be used in feed to improve the growth rate or the feed conversion ratio of animals such as poultry.

Another application resides in the oil extraction where oil has to be extracted from the plant material such as the corn oil from corn embryos. The enzyme with xylanolytic activity of the present invention may be used in fruit and vegetable juice processing to improve the yield. According to the present invention, said enzyme may be used in all processes involving plant materials or waste materials, e.g. from paper production, or agricultural wastes such as wheat-straw, corn cobs, whole corn plants, nut shells, grass, vegetable hulls, bean hulls, spent grains, sugar beet, and the like.

The effect of the enzyme with xylanolytic activity of the present invention may be further improved by adding other enzymes in combination with said enzyme. Such enzymes may belong but are not restricted to hydrolytic enzymes families such as glucanases, proteases, cellulases, hemicellulases, pectinases. Other enzymes are transglutaminases, oxido-reductases, isomerases, etc.

The enzyme with xylanolytic activity according to the invention may be used under several forms. Cells expressing the enzyme, such as yeast, fungi, archea bacteria or bacteria, may be used directly in the process. Said enzyme may be used as a cell extract, a cell-free extract (i.e. portions of the host cell that has been submitted to one or more disruption, centrifugation and/or extraction steps) or as a purified protein. Any of the above-described forms may be used in combination with one or more other enzyme(s) under any of the above-described forms. These whole cells, cell extracts, cell-free extracts or purified enzymes may be immobilised by any conventional means on a solid support to allow protection of the enzyme, continuous hydrolysis of substrate and/or recycling of the enzymatic preparation. Said cells, cell extracts, cell-free extracts or enzymes may be mixed with different ingredients (e.g. in the form of a dry powder or a granulate, in particular a non-dusting granulate, in a form of a liquid, for example with stabilizers such as polyols, sugars, organic acids, sugar alcohols according to well-established methods).

The invention will be described in further details in the following examples by reference to the enclosed drawings, without limiting its scope.

### Brief description of the drawings

Figure 1 shows a SDS-polyacrylamide gel of the proteins recovered after the successive purification steps of the enzyme with xylanolytic activity.

Figure 2 show a Southern blot analysis of the *Penicillium griseofulvum* A160 genomic DNA.

Figure 3 represents the complete genetic sequence of the xylanase according to the invention.

Figure 4 shows the effect of the temperature and of the pH on the xylanase activity.

Figure 5 represents the increase of a bread volume according to the enzymatic activity of the xylanase according to the invention.

### Example 1: Purification of an enzyme with xylanolytic activity from Penicillium griseofulvum A160

### Strain

5 g of a commercial Belgian wheat flour were suspended in 50 ml saline solution (NaCl 0.9%). Aliquots of 100 µl of this suspension were spread on AMAM plates (*Aspergillus* Minimum Agar Medium: glucose 1%, NaNO₃ 0.6%, KCl 7 mM, KH₂PO₄ 11 mM, MgSO₄ 2 mM, ZnSO₄ 76 µM, H₃BO₃ 178 µM, MnCl₂ 25 µM, FeSO₄ 18 µM, CoCl₂ 7.1 µM CuSO₄, 6.4 µM, Na₂MoO₄ 6.2 µM, EDTA 174 µM, pH 6.5 (Pontecorvo et al., 1953, Adv. Genet. Vol 5, p 142) supplemented with 1.5% bacto-agar and 100 µg/ml ampicilline.

Among the strains that appeared on the plates after incubation at 30 °C, a particular strain was isolated and identified as *Penicillium griseofulvum* with the isolation reference A160 (MUCL-41920).

### Determination of the xylanolytic activity

The xylanolytic activity was determined by measuring the reducing sugars formed from the Beechwood xylan (Sigma). The reducing sugars were revealed with the 2,3-dinitrosalicylic acid (Bailey et al., J. Biotechnol. Vol 23, p 257 (1992)). The reaction was carried out at 30°C in a 100 mM acetate buffer at pH 4.5. The xylanolytic activity was expressed in µmole xylose/min.

For rapid identification of the enzyme, the xylanolytic activity was assayed using Azo-xylan (Megazyme) as substrate following the supplier instructions with the exception that the reaction was carried out at 35°C in a 100 mM citrate-phosphate buffer at pH 6.0.

In this case, one xylanase unit was arbitrarily defined as the amount of enzyme required to increase the optical density by one unit at 595 nm in 10 min.

### Purification of the xylanolytic enzyme

The strain of *Penicillium griseofulvum* A160 was cultivated in 2 litres of Aspergillus Minimal Medium pH 6.5 (Ponteverco et al. (1953)), supplemented with 1% xylan from oat spelt (Sigma) at 30°C. After 72 hours, the culture was filtered through a Miracloth filter (Calbiochem) to remove the mycelium. The filtrate was concentrated by ultrafiltration in a Pellicon device with a 10kDa Biomax 10 cassette (Millipore) to a final volume of 170 ml. The concentrate was diluted 3 times to reach a final concentration of 50 mM in sodium acetate pH 4.2.

This solution was loaded at 2 ml/min on a Pharmacia XK16/20 column filled with 30 ml of the Bio-Rad Macro High S resin equilibrated in 50 mM sodium acetate pH 4.2. Proteins were eluted with a linear increasing NaCl gradient from 0 to 0.6 M in 50 mM sodium acetate pH 4.2. Xylanase activity was determined in the eluted fractions. Active fractions were pooled and equilibrated in 1.2 M ammonium sulfate, 50 mM Na acetate pH 5.0 in a final volume of 65 ml.

These were applied on a Phenyl Sepharose HP column (Pharmacia) and eluted at 2.5 ml/min with a 1.2M-OM ammonium sulfate linear gradient in a 50 mM Na acetate pH 5.0 buffer. Xylanase activity was determined in the eluted fractions. The xylanase activity was collected as one peak at 0.8 M ammonium sulfate.

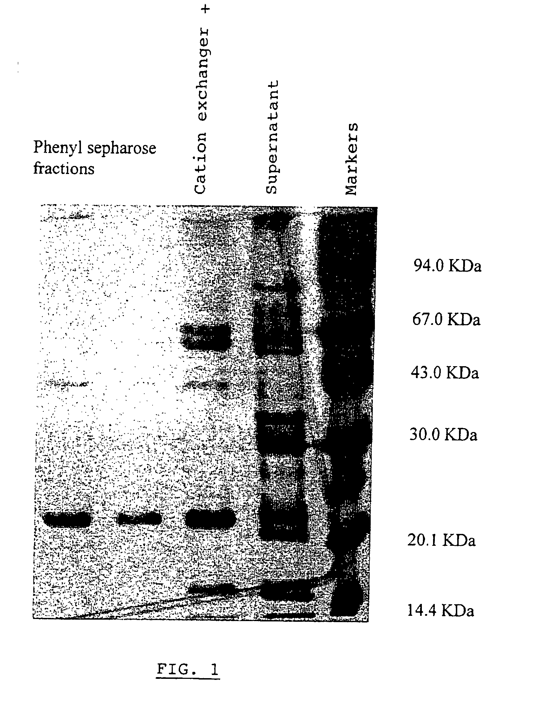

One major protein is present in this peak as shown by SDS-polyacrylamide gel (Figure 1).

### Example 2: Determination of the amino acid sequence of the enzyme with xylanolytic activity

General procedures were followed to perform the N-terminal sequencing of the protein after electrophoresis on a 12% SDS-polyacrylamide gel and electroblotting on a PVDF Immobilon-P membrane (Millipore). An automatic 477A Protein Sequencer coupled to a HPLC 120A Analyser (Applied Biosystem) was used.

The following sequence has been obtained with the protein with an apparent molecular weight of 24 kDa:

### Example 3: Cloning of a gene coding for a enzyme with xylanolytic activity

### Cloning of internal DNA fragments

The genomic DNA from *Penicillium griseofulvum* A160 was isolated according to Boel et al. (EMBO J., vol 7, p. 1581 (1984)). The strain was grown in 50 ml Aspergillus Minimum Medium supplemented with 0.5% Yeast Extract (Difco). After 24 hours, the mycelium was harvested by filtration on a Miracloth filter and washed twice with water. 1 g mycelium was incubated in 10 ml solution A (sorbitol 1 M, EDTA 25 mM, pH 8.0) for 30 min at 30°c. The cells were then centrifuged and suspended in 10 ml solution B (Novozym 234 20 mg, sorbitol 1 M, Na citrate 0.1 M, EDTA 10 mM, pH 5.8). After 30 min at 30°c, the cells were centrifuged and lysed with 15 ml of solution C (phenol 40%, SDS 1%). DNA was separated from the contaminating material by successive extractions with phenol and phenolchloroform, followed by ethanol precipitation.

The degenerated synthetic oligonucleotides mixtures SEQ ID NO 2 and SEQ ID NO 3 were designed based on the N-terminal sequence. A third synthetic oligonucleotides mixture SEQ ID NO 4 has been designed based on a hypothetical degenerated sequence coding for the amino acid sequence EYYIVD, conserved among the family G xylanases.

In these sequences, Y stands for T or C, R for A or G, I for inosine.

The PCR reaction was carried out with 10 ng gDNA of *Penicillium griseofulvum* A160 in the presence of 5 pmole of each synthetic oligonucleotides mixture SEQ ID NO 2 and SEQ ID NO 3 and 10 pmole synthetic oligonucleotides mixture SEQ ID NO 4. The reaction mix contained also 1 unit rTAQ polymerase (Pharmacia), 200µM dNTP, 50 mM KCl, 1.5 mM MgCl2 and 10 mM Tris-HCl pH 9.0 in a final volume of 25 µl. After 4 min of denaturation at 94°c, 25 cycles of [30 s 94°C, 30 s 50°C and 45 s 72 °C] were performed followed by 7 min of elongation at 72°C. Only one fragment of 0.3 kb length was amplified as revealed by agarose gel electrophoresis.

1 µl of the PCR reaction described above was directly sequenced on a ABI 377 sequencer (Applied Biosystem) with either 3 pmoles synthetic oligonucleotides mixtures SEQ ID NO 2 or SEQ ID NO 3 as primers. The sequencing with the oligonucleotides SEQ ID NO 2 and SEQ ID NO 3 gave the nucleotide sequences SEQ ID NO 5 and SEQ ID NO 6, respectively.

An homology search with those sequences against the NCBI proteins database (05-jan-99) using the BLASTX 2.0.8 software found the best homology with the endo-β1,4-xylanase A from *Chaetomium* (accession number: dbj|BAA086491.

### Southern blotting of the Penicillium griseofulvum A160 genomic DNA

Genomic DNA (0.5µg) was digested overnight at 37°C with either 2 units of the restriction enzyme *Eco*RI (Pharmacia), or 2 units of each restriction enzymes *Bam*HI and *Eco*RI (Pharmacia), or 2 units of each restriction enzymes *Eco*RI and *Xba*I in a final volume of 20µl (buffer: 1 x One-Phor-All buffer PLUS (Pharmacia)). The digested DNAs were loaded on a 0.8% agarose gel in 1 x TBE buffer. After electrophoresis, the restricted fragments were transferred onto a Hybond-N+ membrane (Amersham). The PCR fragments described above (1 µl) were labelled with digoxigenin using the DIG High Prime DNA Labelling and Detection Starter Kit II (Boehringer Mannheim). The membrane was hybridised overnight at 42°C in the presence of a standard hybridisation buffer (SSC 5 x, formamide 50%, N-lauroylsarcosine 0.1%, SDS 0.02%, Blocking reagent) and a probe concentration of ca. 10 ng/ml (denatured during 5 min at 97°C). After the hybridisation, the membrane was first washed at 55°C with 2 x SSC, 0.1% SDS (2 x 15 min) followed with 3 washes with a 0.5x SSC, 0.1% SDS solution (30 min). After immunological detection, the hybridising bands were identified by a 4 hours exposition to a Kodak X-OMAT AR film at room temperature.

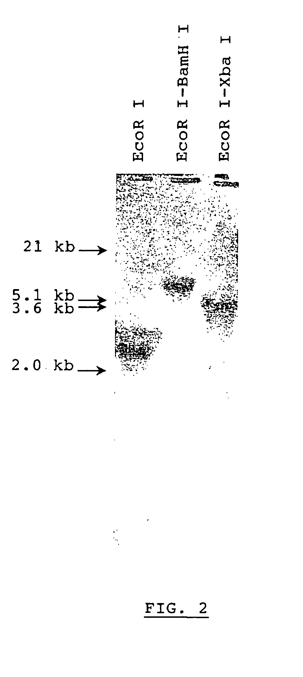

The results of the hybridisation experiment are shown on the figure 2. It revealed that under the hybridisation conditions tested, one DNA fragment hybridised with the probe.

### Construction of a gDNA restriction fragments library of Penicillium griseofulvum A160

Genomic DNA (5µg) was digested overnight at 37°C with 10 units of each restriction enzyme *EcoR*I and *Bam*HI (Pharmacia) in a final volume of 100 µl. The restriction fragments were separated by electrophoresis on a 0.8% agarose gel, 1 x TBE. A piece of the gel corresponding to fragments between 3.5kb and 2.5 kb in length was cutted off. The DNA was purified out of this piece of agarose gel using the Qiaquick gene extraction kit (QIAGEN) in a final volume of 30 µl.

The purified fragments were inserted by ligation between the *Eco*RI and *Bam*HI restriction sites of the pBluescript II SK(+) vector (Stratagene). 1 µg of pBluescript SK(+) plasmid DNA was first digested with 5 units each of *Eco*RI and *Bam*HI restriction enzymes (Pharmacia) in 50 µl (37°C, 16 h) and subsequently purified from both enzymes using the Qiaquick gene extraction kit. The ligation was performed using 3 µl of purified genomic DNA fragments, 0.25 µg of digested pBluescript SK(+) DNA, 3 units of T4 DNA,ligase (Pharmacia), 1 mM ATP in a final volume of 30 µl (1 x One-Phor-All buffer PLUS, 16°C, 16 h). The ligation mixture was then dialysed on a VSWP 013 membrane (Millipore) against water during 20 min. 1 µl of this mixture was electroporated into 40 µl electrocompetent *Escherichia coli* DH10b cells (BRL-Gibco) according to the BRL-Gibco protocol. After electroporation, cells were plated on LB plates supplemented with 100 µg/ml ampicillin to select the transformed cells.

The above-described library was screened progressively using PCR reactions on pools of transformants of decreasing sizes. The PCR reaction conditions were the same as described above with the exception that the template DNA was the plasmids from the pooled *Escherichia coli* transformants purified from 3 ml cultures with the High Pure Plasmid Isolation Kit (Boehringer Mannheim). A 0.3 kb fragment was amplified in one clone out of ca. 1000 clones analysed. The plasmid pPGXYNA recovered from this clone LMBP-3987 contained one *Eco*RI-*Bam*HI insert of 3 kb length. A partial sequence of the pPGXYNA plasmid comprising the xylanolytic enzyme coding sequence was determined on both strands by primer walking using among others the oligonucleotide with the sequence SEQ ID NO 7 as primer.

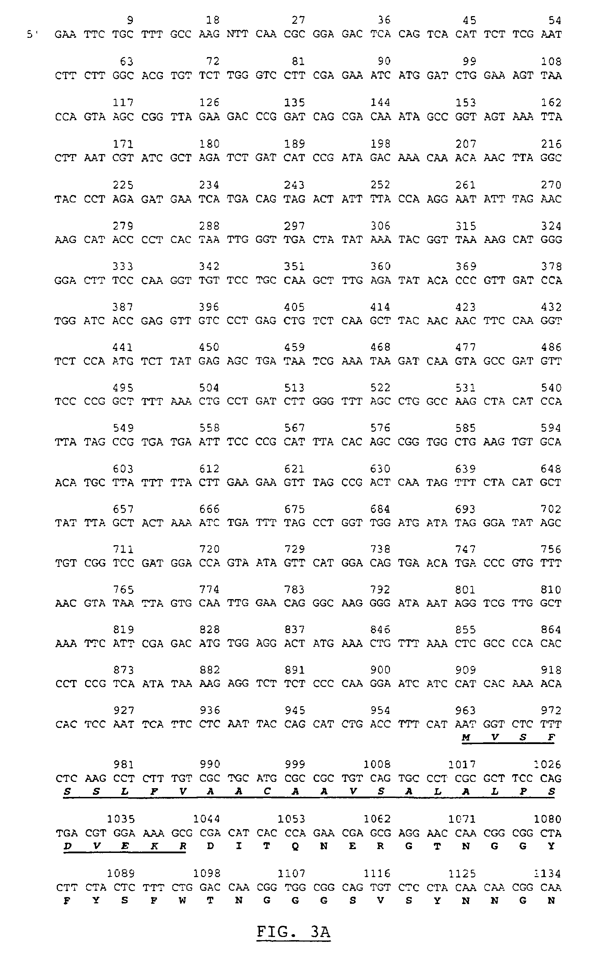

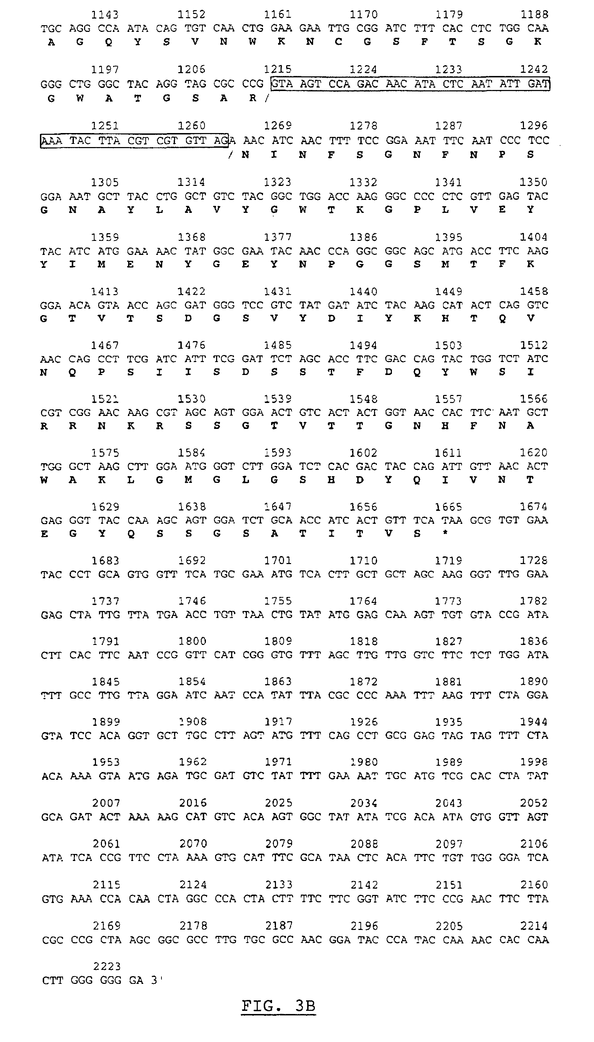

The nucleotide sequence SEQ ID NO 8 according to the invention codes for an amino acid sequence SEQ ID NO 11 and the localisation of an intron was deduced from alignments of the *Penicillium griseofulvum* A160 sequence with the most homologous xylanase protein sequences obtained from a homology search in GENBANK with the BLASTP 2.0.8 software (Altschul et al., Nucl. Ac. Res., vol 25, p 3389 (1997)). This localisation was confirmed by the presence of the putative lariat-formation internal sequence and with the definition of the consensus 5' and 3' splice-junction sequences ('GT-AG' rule). The sequences SEQ ID NO 9 and SEQ ID NO 10 are the sequences encoded by the two exons of the enzyme with xylanolytic activity. The sequence SEQ ID NO 11 is the amino acid sequence of the *Penicillium griseofulvum* A160 enzyme. A signal sequence driving the secretion of the enzyme covers the 27 first amino acids of the sequence (Fig. 3).

### Example 4: Expression of the xylanolytic enzyme gene in Aspergillus oryzae

### Construction of expression vectors

A DNA fragment covering the coding region as well as its terminator region was amplified by PCR. The first synthetic oligonucleotide SEQ ID NO 12 was chosen to contain the ATG codon corresponding to the first methionine of the coding region of the polypeptide gene as well as a recognition site for the restriction enzyme *Eco*RI. The second oligonucleotide SEQ ID NO 13 corresponded to the sequence located 250 bp downstream of the last codon and contained a XbaI restriction site.

Both primers (40 pmoles) were used for a PCR reaction with ca. 40 ng of pPGXYNA plasmid DNA as template. The 100 µl PCR reaction contained also 2.5 units *Pfu* DNA polymerase (Stratagene) and 1 µg BSA in the following buffer: Tris-HCl pH8.0 20 mM, KCl 10 mM, MgCl₂ 2 mM, (NH4)₂SO₄ 6 mM and Triton X-100 0.1%. After denaturation of the DNA during 4 min at 94°C, 20 cycles of elongation were performed [30s at 94°C, 30s at 55°C and 60s at 72°C] followed by an elongation step of 7 min at 72°C. The amplified DNA fragment was purified with the QiaQuick PCR purification kit (Qiagen) according to the manufacturer's protocol and recovered in a final volume of 50 µl. The extremities of the fragment were removed by digestion with the *Eco*RI and *Xba*I restriction enzymes (5 units of *Xba*I and 5 units of *Eco*RI enzymes (Pharmacia), 1 x One-Phor-All buffer PLUS, final volume 60 µl, 37°C, overnight). The fragment was then purified with the QIAquick gel extraction kit (Qiagen) after separation by electrophoresis on an agarose gel and recovered in 30 µl water.

The PCR DNA fragment was inserted between the *Eco*RI and *Xba*I restriction sites of the pBluescript II SK(+) vector (Stratagene). The vector was prepared as follows: 0.5 µg pBluescript SK(+) DNA was digested with 5 units EcoRI and 5 units XbaI restriction enzymes (Pharmacia) (final volume 20 µl, 2x One-Phor-All buffer PLUS, 37°C, overnight). After separation by electrophoresis in an agarose gel, it was purified with the QIAquick gel extraction kit (Qiagen) and recovered in 30 µl water.

2 µl of PCR DNA fragment were ligated with this vector (1 µl) in the presence of ATP (1 mM), 1 unit of T4 DNA ligase (Pharmacia) and 1x One-Phor-All buffer PLUS (final volume 10 µl, 16°C, overnight). 1 µl of the ligation mixture was electroporated in competent *Escherichia coli* DH10b cells (BRL-Gibco) after dialysis against water. A right clone was selected after analysis of a number of transformants plasmids by extraction, digestion with appropriate restriction enzymes and separation by electrophoresis on an agarose gel using standard procedures. The new plasmid was termed pPGXYN1E-X.

The promoter of the glyceraldehyde-3-P dehydrogenase gene from *Aspergillus nidulans* was cloned in front of the xylanolytic enzyme gene. This promoter allows a strong constitutive transcription of the genes located downstream of it. (Punt *et al*., 1990, Gene, vol 93, p 101; Punt *et al.,* 1991, J. Biotechnol., vol 17, p 19). The plasmid pFGPDGLAT2 contains this promoter between two restriction sites: *Eco*RI and *Nco*I. This promoter was inserted into the pBluescript II SK(+) plasmid between two EcoRI restriction sites to give the pSK-GPDp plasmid using standard procedures. This plasmid (1 µg) as well as pPGXYN1E-X (1µg) were digested by the *EcoR*I restriction enzyme (5 units) in the presence of 1x One-Phor-All buffer PLUS (final volume 10 µl, 37°C, overnight). The DNA fragments of interest were then separated by electrophoresis on an agarose gel and purified with the QIAquick gel extraction kit (Qiagen) and collected in 30 µl water. The purified promoter DNA fragment (1 µl) was inserted by ligation between the *EcoR*I recognition sites of pPGXYN1E-X (1µl) in the presence of ATP (1 mM), 1 unit of T4 DNA ligase (Pharmacia) and 1 x One-Phor-All buffer PLUS (final volume 10 µl, 16°C, overnight). 1 µl of the ligation mixture was electroporated into competent *Escherichia coli* DH10b cells (BRL-Gibco) after dialysis against water. A right clone was selected after analysis of a number of transformants plasmids by extraction, digestion with appropriate restriction enzymes and separation by electrophoresis on an agarose gel using standard procedures. The new plasmid was termed pGPDp-PGXYN1.

### Transformation of Aspergillus oryzae

The strain *Aspergillus oryzae* MUCL 14492 was transformed by generating protoplasts according to the protocol described by Punt et al. (Meth. Enzymol, vol 216, p 447 (1992)). The pGPDp-PGXYN1 plasmid was cotransformed with the p3SR2 plasmid that contains a selection marker used to recover transformants (the *Aspergillus nidulans* acetamidase gene - Hynes et al., Mol. Cell. Biol., vol 3, p 1430 (1983))., Transformants were selected on minimum medium plates containing acetamide as sole nitrogen source.

The strain *Aspergillus oryzae* MUCL 14492 was grown in 500 ml Aspergillus Minimum Liquid medium (Pontecorvo et al. (1992)) during 16 hours at 30°C. The culture was filtered through a Miracloth filter to collect the mycelium. The mycelium was washed with the Osm solution (CaCl₂ 0.27 M, NaCl 0.6 M). and then incubated with 20 ml solution Osm/g mycelium supplemented with 20 mg Novozym 234 (Sigma). After 1 hour at 30°C with slow agitation (80 rpm), the protoplasts were formed and the suspension was putted on ice. The protoplasts were separated from intact mycelium by filtration through a sterile Miracloth filter and diluted with 1 volume STC1700 solution (sorbitol 1.2 M, Tris-HCl pH 7.5 10 mM, CaCl₂ 50 mM, NaCl 35 mM). The protoplasts were then collected by centrifugation at 2000 rpm, 10 min, 4°C and washed twice with STC1700 solution. They were resuspended in 100 µl of STC1700 (10⁸ protoplasts/ml) in the presence of 3 µg p3SR2 plasmid DNA and 9 µg pGPDp-PGXYN1 plasmid DNA. After 20 min at 20°C, 250, 250 and 850 µl PEG solution (PEG 4000 60%, Tris-HCl pH 7. 5 10 mM and CaCl₂ 50 mM) were added successively and the suspension was further incubated for 20 min at 20°C. PEG treated protoplast suspensions were diluted by the addition of 10 ml STC1700 and centrifugated 10 min at 4°C, 2000 rpm. The protoplasts were then resuspended in 200 µl STC 1700 and plated onto Aspergillus Minimum Agar Medium osmotically stabilized with 1.2 M sorbitol. To select the transformants, the nitrogen sources in the plates were replaced by 10 mM acetamide and 12 mM CsCl.

### Analysis of Aspergillus oryzae transformants

48 transformants were analysed for the xylanolytic enzyme expression. They were grown in Aspergillus Minimum Liquid Medium supplemented with 3% sucrose as carbon source and 0.5% Bacto yeast extract (Difco). After 75 hours at 30°C and 130 rpm, the supernatant of the cutures was assayed for xylanolytic activity. 10 of the transformants showed a significantly higher xylanolytic activity as compared to a control strain transformed only with the p3SR2 plasmid.

### Example 5: Characterisation of the enzyme with xylanolytic activity from Penicillium griseofulvum A160

### Purification of the enzyme with xylanolytic activity expressed in Aspergillus oryzae

The enzyme with xylanolytic activity expressed in *Aspergillus oryzae* was purified in order to separate it from the traces of α-amylase present in the culture supernatants of the transformants. 10 ml of a culture supernatant from a selected transformant were diluted 3 times to reach a final concentration of 50 mM in sodium acetate pH 4.2.

This solution was loaded at 2 ml/min on a Pharmacia XK16/20 column filled with c.a. 30 ml of the Bio-Rad Macro High S resin equilibrated in 50 mM sodium acetate pH 4.2. Proteins were eluted with a linear increasing NaCl gradient from 0 to 0.6 M in 50 mM sodium acetate pH 4.2. Xylanase and amylase activities were determined in the eluted fractions. The amylase activity was recovered in the flow through fractions while the xylanase activity was eluted approximately at 0.1 M NaCl. The active fractions with xylanolytic activity were pooled and kept for further analysis.

### Optimum pH and temperature

The pH and temperature dependance of the activity of the xylanolytic enzyme secreted by one *Aspergillus oryzae* transformant was analysed. The activity was measured in a citrate/phosphate buffer (0.1M) at various pH (Fig. 4).

The maximum activity was observed around 50°C. At this temperature, the optimum pH was about 5.0.

These properties are similar to those of the enzyme with xylanolytic activity purified from the *Penicillium griseofulvum* A160.

### Example 6: Baking trials

Baking trials were performed to demonstrate the positive effect of the *Aspergillus griseofulvum* A160 xylanase in baking. The positive effect was evaluated by the increase in bread volume compared to a reference not containing the enzyme.

The xylanase was tested in Belgian hard rolls that are produced on a large scale every day in Belgium. The procedure described is well known to the craft baker and it is obvious to one skilled in the art that the same results may be obtained by using equipment from other suppliers.

The ingredients used are listed in the table below:

| **Ingredients (g)** | **RECIPE 1** | **RECIPE 2** | **RECIPE 3** | **RECIPE 4** | **RECIPE 5** |
|---|---|---|---|---|---|
| Flour (Surbi - Molens van Deinze) | 1500 | 1500 | 1500 | 1500 | 1500 |
| Water | 915 | 915 | 915 | 915 | 915 |
| Fresh yeast (Bruggeman - Belgium) | 90 | 90 | 90 | 90 | 90 |
| Sodium chloride | 30 | 30 | 30 | 30 | 30 |
| Ascorbic acid | 0.12 | 0.12 | 0.12 | 0.12 | 0.12 |
| Multec Data 2720S™ | 3.5 | 3.5 | 3.5 | 3.5 | 3.5 |
| Dextrose | 10 | 10 | 10 | 10 | 10 |
| Xylanase™ A160 (Megazyme units) | 0 | 23 | 35 | 52 | 70 |

The ingredients were mixed for 2 min at low and 7 min at high speed in a Diosna SP24 mixer. The final dough temperature as well as the resting and proofing temperatures were 25°C. After resting for 15 min at 25°C, the dough was reworked manually and rested for another 10 min. Afterwards, 2 kg dough pieces were made up and proofed for 10 min. The 2 kg dough pieces were divided and made up using the Eberhardt Optimat. 66 gr round dough pieces were obtained. After another 5 min resting time, the dough pieces were cut by pressing and submitted to a final proofing stage for 70 min.

The dough pieces were baked at 230°C in a Miwe Condo™ oven with steam (Michael Wenz - Arnstein - Germany). The volume of 6 rolls was measured using the commonly used rapeseed displacement method.

The results are presented on the table below:

| Xylanase units | Volume (ml) |
|---|---|
| 0 | 2125 |
| 23 | 2475 |
| 35 | 2550 |
| 52 | 2675 |
| 70 | 2775 |

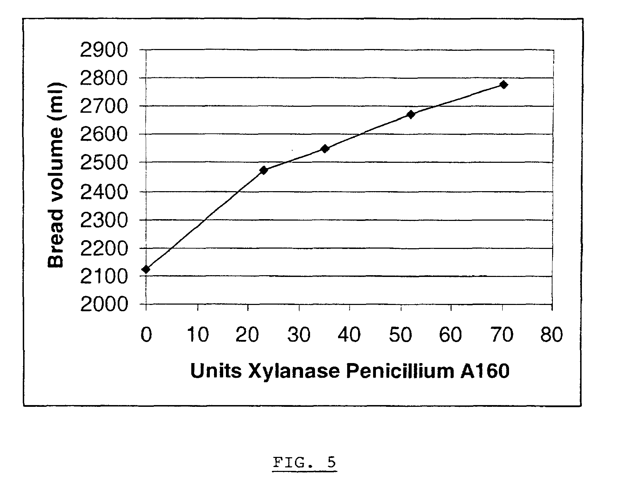

A graphical representation of the effect of the xylanase on bread volume is shown on figure 5.

### Example 7: Effect of the enzyme on the flour viscosity in the presence of water

Purified xylanase was used for the test. The enzyme was purified as described in example 5. 100 g of wheat flour (Surbi, Molens van Deinze) were mixed manually with 117 ml water containing 25 xylanase units of the enzyme with xylanolytic activity from *Penicillium griseofulvum* A160. After 15 min at 35°C, the viscosity was measured (Programmable DV-II + Viscometer, Helipath system, Spindel F, Brookfield). The speed was maintained at 4 rpm and the viscosity value was measured after 10 s. The viscosity of a blank sample was obtained in the same way with untreated flour. The same experiment was also carried out with 10 units of the best performing enzyme available actually on the market *(Aspergillus aculeatus* xylanase available from Novo Nordisk (Shearzyme™ L)). Each experiment was performed in triplicate. The viscosity results presented below are expressed in centipoises.

| | |
|---|---|
| Blank sample | 116.000 +/- 2000 |
| *Penicillium griseofulvum* enzyme | 65.034 +/- 5047 |
| *Aspergillus aculeatus* xylanase | 68.959 +/- 2253 |

*Aspergillus aculeatus* xylanase was shown to give better results than xylanases from *Humicola insolens*, *Trichoderma reesei* (Spezyme CP, Genencor) and another xylanase from *Aspergillus aculeatus* (xylanase I) (patent application WO 94/21785). Christophersen *et al.* (Christophersen *et al,* 1997, Starch/Stärke, vol 49, p 5)) also showed the better performance of *Aspergillus aculeatus* xylanase as compared to a xylanase from *Thermomyces lanuginosus* and two commercial hemicellulase cocktails sold for wheat separation.

The results presented above showed that the enzyme with xylanolytic activity from *Penicillium griseofulvum* A160 has the biggest capacity of reducing the viscosity of flour suspended in water.

### Example 8: Wheat separation

When mixed with water, the flour may be separated into a starch, a gluten, a sludge and a soluble fraction by centrifugation. A decrease of the sludge fraction leads to a better wheat separation. The performances of a pure xylanase could therefore be evaluated by measuring the decrease of the solid sludge fraction after centrifugation.

Such experiment has been carried out with the purified enzyme with xylanolytic activity from *Penicillium griseofulvum* A160 of example 5 compared to Shearzyme™ L.

100 g of wheat flour (Surbi, Molens of Deinze) were mixed manually with 117 ml water containing different concentrations of enzyme. After 15 min at 35°C, the mixture was centrifugated for 10 min at 4000 g (Varifuge 3.0R, Heraeus Sepatech). The liquid phase was weighted.

The table below shows the results of a typical experiment, by reporting the relative increase of the liquid phase induced by the presence of the xylanolytic enzyme. The enzyme from *Penicillium griseofulvum* A160 allowed to reach a higher liberation of liquid than Shearzyme™ L.

| **Enzyme (units/test)** | **P. griseofulvum enzyme (%)** | **Shearzyme (%)** |
|---|---|---|
| 0 | 100 | 100 |
| 3.125 | 110 | 127 |
| 6.25 | 129 | 130 |
| 12.5 | 134 | 134 |
| 25 | 166 | 137 |

The applicant has made a deposit of micro-organism for the strain *Penicillium griseofulvum* Diercks A160 according to the invention under the deposit number MUCL 41920 on 13/12/1999 at the BCCM/MUCL Culture Collection (Mycothèque de l'Université Catholique de Louvain, Place de la Croix du Sud 3, B-1348 LOUVAIN-LA-NEUVE, BELGIUM and the deposit of the micro-organism *Escherichia coli* DH10B (pPGXYNA) according to the invention on 13/12/1999 under the deposit number LMBP 3987 at the Laboratorium voor Moleculaire Biologie BCCM/LMBP (K.L. Ledeganckstraat 35, B-9000 GENT, BELGIUM).

## Claims

1. An isolated and purified enzyme with xylanolytic activity having more than 80% sequence identity over the entire length with the amino acid sequence SEQ ID NO 11.

2. The enzyme according to claim 1, having more than 90% sequence identity over the entire length with the amino acid sequence SEQ ID NO 11.

3. An isolated and purified enzyme with xylanolytic activity with the amino acid sequence of SEQ ID NO:11.

4. An isolated and purified enzyme with xylanolytic activity having the amino acid sequence DITQNERGTNNGYFYSFWTXGGGNYV at its N-terminal end.

5. The enzyme according to any of the preceding claims, **characterised in that** it presents an optimum enzymatic activity at a pH between 4.5 and 7.0 and at a temperature comprised between 35 and 55 °C.

6. An isolated and purified nucleotide sequence encoding the enzyme according to any one of the preceding claims.

7. An isolated and purified nucleotide sequence which encodes a polypeptide having a xylanolytic activity, **characterised in that** it has more than 80% sequence identity over the entire length with the nucleotide sequence SEQ ID NO 8.

8. The isolated and purified nucleotide sequence according to claim 7, **characterised in that** it has more than 90% sequence identity over the entire length with the nucleotide sequence SEQ ID NO 8.

9. An isolated and purified nucleotide sequence SEQ ID NO 8.

10. A recombinant nucleotide sequence comprising, operably linked to the nucleotide sequence according to any one of the claims 6 to 9, one or more adjacent regulatory sequence(s).

11. A recombinant nucleotide sequence according to the claim 10, wherein the adjacent regulatory sequence is originating from homologous micro-organisms.

12. A vector comprising the nucleotide sequence according to any one of the claims 6 to 11.

13. The vector according to claim 12, being a plasmid incorporated in *Escherichia coli* and having the deposit number LMBP-3987.

14. A recombinant host cell transformed by the nucleotide sequence according to any one of the claims 5 to 11 or by the vector according to claim 12 or 13.

15. The recombinant host cell according to claim 14, **characterised in that** it is selected from the group consisting of bacteria or fungi, including yeast.

16. A solid support fixing an element selected from the group consisting of the cell according to claim 14 or 15, of a cell extract of the cell according to claim 14 or 15containing the enzyme with xylanolytic activity according to any one of the claims 1 to 5 and/or of the isolated and purified enzyme with xylanolytic activity according to any one of the claims 1 to 5.

17. Use of the recombinant host cell according to claim 14 or 15, expressing the enzyme with xylanolytic activity according to any one of the claims 1 to 5, or of the enzyme with xylanolytic activity according to any one of the claims 1 to 5 for the degradation of plant cell wall components.

18. Use according to claim 17 in processes for decomposing plants and fruits, preferably fruit, legume juices, beer, paper, starch, gluten or vegetable oil preparation processes.

19. Use according to claim 17 in processes for decomposing wastes, preferably for decomposing agricultural wastes or wastes from paper mills.

20. Use according to claim 17 in baking processes, especially for increasing the volume of baked products.

21. Use according to claim 17 in starch-gluten separation processes.

## Patentansprüche

1. Isoliertes und gereinigtes Enzym mit xylanolytischer Aktivität mit mehr als 80 % Sequenzidentität über die gesamte Länge mit der Aminosäuresequenz SEQ ID NO 11.

2. Enzym nach Anspruch 1 mit mehr als 90 % Sequenzidentität über die gesamte Länge mit der Aminosäuresequenz SEQ ID NO 11.

3. Isoliertes und gereinigtes Enzym mit xylanolytischer Aktivität mit der Aminosäuresequenz SEQ ID NO 11.

4. Isoliertes und gereinigtes Enzym mit xylanolytischer Aktivität mit der Aminosäuresequenz DITQNERGTNNGYFYSFWTXGGGNYV an seinem N-terminalen Ende.

5. Enzym nach irgendeinem der vorhergehenden Ansprüche, **dadurch gekennzeichnet, dass** es eine optimale enzymatische Aktivität bei einem pH-Wert zwischen 4,5 und 7,0 und bei einer Temperatur aufweist, die zwischen 35 und 55 °C liegt.

6. Isolierte und gereinigte Nukleotidsequenz, die das Enzym nach irgendeinem der vorhergehenden Ansprüche kodiert.

7. Isolierte und gereinigte Nukleotidsequenz, die ein Polypeptid mit xylanolytischer Aktivität kodiert, **dadurch gekennzeichnet, dass** sie mehr als 80 % Sequenzidentität über die gesamte Länge mit der Nukleotidsequenz SEQ ID NO 8 hat.

8. Isolierte und gereinigte Nukleotidsequenz nach Anspruch 7, **dadurch gekennzeichnet, dass** sie mehr als 90 % Sequenzidentität über die gesamte Länge mit der Nukleotidsequenz SEQ ID NO 8 hat.

9. Isolierte und gereinigte Nukleotidsequenz SEQ ID NO 8.

10. Rekombinante Nukleotidsequenz, die eine oder mehrere benachbarte Regulationssequenz(en) umfasst, die funktionsfähig mit der Nukleotidsequenz nach irgendeinem der Ansprüche 6 bis 9 verbunden sind.

11. Rekombinante Nukleotidsequenz nach Anspruch 10, wobei die benachbarte Regulationssequenz aus homologen Mikroorganismen stammt.

12. Vektor, der die Nukleotidsequenz nach irgendeinem der Ansprüche 6 bis 11 umfasst.

13. Vektor nach Anspruch 12, der ein in *Escherichia coli* eingebautes Plasmid ist und die Hinterlegungsnummer LMBP-3987 hat.

14. Rekombinante Wirtszelle, die durch die Nukleotidsequenz nach irgendeinem der Ansprüche 5 bis 11 oder den Vektor nach Anspruch 12 oder 13 transformiert ist.

15. Rekombinante Wirtszelle nach Anspruch 14, **dadurch gekennzeichnet, dass** sie aus der Gruppe bestehend aus Bakterien und Pilzen einschließlich Hefen ausgewählt ist.

16. Fester Träger, der ein Element ausgewählt aus der Gruppe bestehend aus der Zelle nach Anspruch 14 oder 15, einem Zellextrakt der Zelle nach Anspruch 14 oder 15, die das Enzym mit xylanolytischer Aktivität nach irgendeinem der Ansprüche 1 bis 5 enthält, und/oder dem isolierten und gereinigten Enzym mit xylanolytischer Aktivität nach irgendeinem der Ansprüche 1 bis 5 fixiert.

17. Verwendung der rekombinanten Wirtszelle nach Anspruch 14 oder 15, die das Enzym mit xylanolytischer Aktivität nach irgendeinem der Ansprüche 1 bis 5 exprimiert, oder des Enzyms mit xylanolytischer Aktivität nach irgendeinem der Ansprüche 1 bis 5 zum Abbau von Pflanzenzellwandkomponenten.

18. Verwendung nach Anspruch 17 in Verfahren zum Zersetzen von Pflanzen und Früchten, insbesondere in Frucht-, Hülsenfruchtsäfte-, Bier-, Papier-, Stärke-, Glutenoder Pflanzenölherstellungsverfahren.

19. Verwendung nach Anspruch 17 in Verfahren zum Zersetzen von Abfällen, vorzugsweise zum Zersetzen von Abfällen der Landwirtschaft oder Abfällen aus Papiermühlen.

20. Verwendung nach Anspruch 17 in Backverfahren, insbesondere zum Erhöhen des Volumens von gebackenen Produkten.

21. Verwendung nach Anspruch 17 in Stärke-Gluten-Trennverfahren.

## Revendications

1. Enzyme isolée et purifiée ayant une activité xylanolytique ayant une identité de séquence de plus de 80% sur toute la longueur avec la séquence d'acides aminés SEQ ID n° 11.

2. Enzyme selon la revendication 1 ayant une identité de séquence de plus de 90% sur toute la longueur avec la séquence d'acides aminés SEQ ID n° 11.

3. Enzyme isolée et purifiée ayant une activité xylanolytique avec la séquence d'acides aminés SEQ ID n° 11.

4. Enzyme isolée et purifiée ayant une activité xylanolytique ayant la séquence d'acides aminés DITQNERGTNNGYFYSFWTXGGGNYV à son extrémité N-terminale.

5. Enzyme selon l'une quelconque des revendications précédentes, **caractérisée en ce qu'**elle présente une activité enzymatique optimale à un pH compris entre 4,5 et 7,0 et à une température comprise entre 35 et 55°C.

6. Séquence de nucléotides isolée et purifiée codant pour l'enzyme selon l'une quelconque des revendications précédentes.

7. Séquence de nucléotides isolée et purifiée codant pour un polypeptide ayant une activité xylanolytique, **caractérisée en ce qu'**elle a une identité de séquence de plus de 80% sur toute la longueur avec la séquence de nucléotides SEQ ID n° 8.

8. Séquence de nucléotides isolée et purifiée selon la revendication 7, **caractérisée en ce qu'**elle a une identité de séquence de plus de 90% sur toute la longueur avec la séquence de nucléotides SEQ ID n° 8.

9. Séquence de nucléotides isolée et purifiée SEQ ID n° 8.

10. Séquence de nucléotides recombinante comprenant une ou plusieurs séquences régulatrices adjacentes liées fonctionnellement à la séquence de nucléotides selon l'une quelconque des revendications 6 à 9.

11. Séquence de nucléotides recombinante selon la revendication 10, dans laquelle la séquence régulatrice adjacente provient de micro-organismes homologues.

12. Vecteur comprenant la séquence de nucléotides selon l'une quelconque des revendications 6 à 11.

13. Vecteur selon la revendication 12, qui est un plasmide incorporé à la souche *Escherichia coli* et qui a le numéro de dépôt LMBP-3987.

14. Cellule hôte recombinante transformée par la séquence de nucléotides selon l'une quelconque des revendications 5 à 11 ou par le vecteur selon la revendication 12 ou 13.

15. Cellule hôte recombinante selon la revendication 14, **caractérisée en ce qu'**elle est choisie dans le groupe constitué par des bactéries ou des champignons, notamment la levure.

16. Support solide fixant un élément choisi dans le groupe constitué de la cellule selon la revendication 14 ou 15, d'un extrait cellulaire de la cellule selon la revendication 14 ou 15 contenant l'enzyme ayant une activité xylanolytique selon l'une quelconque des revendications 1 à 5 et/ou de l'enzyme isolée et purifiée ayant une activité xylanolytique selon l'une quelconque des revendications 1 à 5.

17. Utilisation de la cellule hôte recombinante selon la revendication 14 ou 15, exprimant l'enzyme ayant une activité xylanolytique selon l'une quelconque des revendications 1 à 5, ou de l'enzyme ayant une activité xylanolytique selon l'une quelconque des revendications 1 à 5 pour la dégradation de composants de paroi cellulaire de plantes.

18. Utilisation selon la revendication 17 dans des procédés pour la décomposition de plantes et de fruits, de préférence des procédés de préparation de jus de fruits, de jus de légumineuses, de bière, de papier, d'amidon, de gluten ou d'huile végétale.

19. Utilisation selon la revendication 17 dans des procédés pour la décomposition de déchets, de préférence pour la décomposition de déchets agricoles ou de déchets provenant d'usines à papier.

20. Utilisation selon la revendication 17 dans des procédés de cuisson, en particulier pour augmenter le volume de produits cuits.

21. Utilisation selon la revendication 17 dans des procédés de séparation amidon-gluten.


### SEQUENCE LISTING

<110> PURATOS N.V.
<120> ENZYME WITH XYLANASE ACTIVITY
<130> P.PURA.10/EP
<140>
<141>
<160> 13
<170> PatentIn Ver. 2.1
<210> 1
<211> 26
<212> PRT
<213> Penicillium griseofulvum
<400> 1
   Asp Ile Thr Gln Asn Glu Arg Gly Thr Asn Asn Gly Tyr Phe Tyr Ser
     1               5                  10                  15
   Phe Trp Thr Xaa Gly Gly Gly Asn Val Tyr
                20                  25
<210> 2
<211> 23
<212> DNA
<213> Artificial Sequence
<220>
<223> Description of Artificial Sequence: synthetic oligonucleotide
<400> 2
   ggytayttyt ayaayttytg gac 23
<210> 3
<211> 23
<212> DNA
<213> Artificial Sequence
<220>
<223> Description of Artificial Sequence: synthetic oligonucleotide
<220>
<221> modified_base
<222> (15)
<223> i
<400> 3
<210> 4
<211> 17
<212> DNA
<213> Artificial Sequence
<220>
<223> Description of Artificial Sequence: synthetic oligonucleotide
<400> 4
   tcgacraygt agtaytc 17
<210> 5
<211> 102
<212> DNA
<213> Penicillium griseofulvum
<400> 5
   cnagtacaac aacgnnaagn ccggcnaata cagngtgnan tggaagaact gcggntattt 60
   cacctctggc aagggctgga nnactggtag ngcccggtaa gt                    102
<210> 6
<211> 87
<212> DNA
<213> Penicillium griseofulvum
<400> 6
   cggcnaatac aagggtgtna ntggaagaac tgcggnnatt tcacctcngg caagggctgg 60
   actactggta gtgcccggta agtgcaa                                     87
<210> 7
<211> 22
<212> DNA
<213> Artificial Sequence
<220>
<223> Description of Artificial Sequence: synthetic oligonucleotide
<400> 7
   tatttcacct ctggcaaggg ct                                          22
<210> 8
<211> 222,5
<212> DNA
<213> Penicillium griseofulvum
<220>
<221> exon
<222> (962)..(1213)
<220>
<221> intron
<222> (1214)..(1261)
<220>
<221> exon
<222> (1263)..(1661)
<220>
<221> CDS
<222> (962)..(1213)
<220>
<221> CDS
<222> (1264)..(1662)
<400>
   gaattctgct ttgccaagnt tcaacgcgga gactcacagt cacattcttc gaatcttctt 60
   ggcacgtgtt cttgggtcct tcgagaaatc atggatctgg aaagttaacc agtaagccgg 120
   ttagaagacc cggatcagcg acaaatagcc ggtagtaaat tacttaatcg tatcgctaga 180
   tctgatcatc cgatagacaa acaaacaaac ttaggctacc ctagagatga atcatgacag 240
   tagactattt taccaaggaa tatttagaac aagcataccc ctcactaatt gggttgacta 300
   tataaatacg gttaaaagca tgggggactt tcccaaggtt gttcctgcca agctttgaga 360
   tatacacccg ttgatccatg gatcaccgag gttgtccctg agctgtctca agcttacaac 420
   aacttccaag gttctccaat gtcttatgag agctgataat cgaaataaga tcaagtagcc 480
   gatgtttccc cggcttttaa actgcctgat cttgggttta gcctggccaa gctacatcca 540
   ttatagccgt gatgaatttc cccgcattta cacagccggt ggctgaagtg tgcaacatgc 600
   ttatttttac ttgaagaagt ttagccgact caatagtttc tacatgctta tttagctact 660
   aaaatctgat tttagcctgg ttggatgata tagggatata gctgtcggtc cgatggacca 720
   gtaatagttc atggacagtg aacatgaccc gtgtttaacg tataattagt gcaattggaa 780
   cagggcaagg ggataaatag gtcgttggct aaattcattc gagacatgtg gaggactatg 840
   aaactgttta aactcgcccc acaccctccg tcaatataaa agaggtcttc tccccaagga 900
   atcatccatc acaaaacaca ctccaattca ttcctcaatt accagcatct gacctttcat 960
   a atg gtc tct ttc tca agc ctc ttt gtc gct gca tgc gcc gct gtc agt 1009
     Met Val Ser Phe Ser Ser Leu Phe Val Ala Ala Cys Ala Ala Val Ser
     1               5                   10                  15
   gcc ctc gcg ctt ccc agt gac gtg gaa aag cgc gac atc acc cag aac   1057
   Ala Leu Ala Leu Pro Ser Asp Val Glu Lys Arg Asp Ile Thr Gln Asn
               20                  25                  30
   gag cga gga acc aac ggc ggc tac ttc tac tct ttc tgg acc aac ggt   1105
   Glu Arg Gly Thr Asn Gly Gly Tyr Phe Tyr Ser Phe Trp Thr Asn Gly
           35                  40                  45
   ggc ggc agt gtc tcc tac aac aac ggc aat gca ggc caa tac agt gtc   1153
   Gly Gly Ser Val Ser Tyr Asn Asn Gly Asn Ala Gly Gln Tyr Ser Val
       50                  55                  60
   aac tgg aag aat tgc gga tct ttc acc tct ggc aag ggc tgg gct aca   1201
   Asn Trp Lys Asn Cys Gly Ser Phe Thr Ser Gly Lys Gly Trp Ala Thr
   65                  70                  75                  80
   ggt agc gcc cgg taagtccaga caacatactc aatattgata aatacttacg       1253
   Gly Ser Ala Arg
   tcgtgttaga aac atc aac ttt tcc gga aat ttc aat ccc tcc gga aat    1302
              Asn Ile Asn Phe Ser Gly Asn Phe Asn Pro Ser Gly Asn
               85                  90                  95
   gct tac ctg gct gtc tac ggc tgg acc aag ggc ccc ctc gtt gag tac   1350
   Ala Tyr Leu Ala Val Tyr Gly Trp Thr Lys Gly Pro Leu Val Glu Tyr
           100                 105                 110
   tac atc atg gaa aac tat ggc gaa tac aac cca ggc ggc agc atg acc    1398
   Tyr Ile Met Glu Asn Tyr Gly Glu Tyr Asn Pro Gly Gly Ser Met Thr
       115                 120                 125
   ttc aag gga aca gta acc agc gat ggg tcc gtc tat gat atc tac aag    1446
   Phe Lys Gly Thr Val Thr Ser Asp Gly Ser Val Tyr Asp Ile Tyr Lys
   130                 135                 140                 145
   cat act cag gtc aac cag cct tcg atc att tcg gat tct agc acc ttc    1494
   His Thr Gln Val Asn Gln Pro Ser Ile Ile Ser Asp Ser Ser Thr Phe
                   150                 155                 160
   gac cag tac tgg tct atc cgt cgg aac aag cgt agc agt gga act gtc    1542
   Asp Gln Tyr Trp Ser Ile Arg Arg Asn Lys Arg Ser Ser Gly Thr Val
               165                 170                 175
   act act ggt aac cac ttc aat gct tgg gct aag ctt gga atg ggt ctt    1590
   Thr Thr Gly Asn His Phe Asn Ala Trp Ala Lys Leu Gly Met Gly Leu
           180                 185                 190
   gga tct cac gac tac cag att gtt aac act gag ggt tac caa agc agt    1638
   Gly Ser His Asp Tyr Gln Ile Val Asn Thr Glu Gly Tyr Gln Ser Ser
       195                 200                 205
   gga tct gca acc atc act gtt tca taagcgtgtg aataccctgc agtggtttca  1692
   Gly Ser Ala Thr Ile Thr Val Ser
   210                 215
   tgcgaaatgt cacttgctgc tagcaagggt ttggaagagc tattgttatg aacctgttaa 1752
   ctgtatatgg agcaaagttg tgtaccgata cttcacttca atccggttca tcgggtgttt 1812
   agcttgttgg tcttctcttg gatatttgcc ttgttaggaa tcaatccata tttacgcccc 1872
   aaatttaagt ttctaggagt atccacaggt gcttgcctta gtatgtttca gcctgcggag 1932
   tagtagtttc taacaaaagt aatgagatgc gatgtctatt ttgaaaattg catgtcgcac 1992
   ctatatgcag atactaaaaa gcatgtcaca agtggctata tatcgacaat agtggttagt 2052
   atatcaccgt tcctaaaagt gcatttcgca taactcacat tctgttgggg atcagtgaaa 2112
   ccacaactag gcccactact tttcttcggt atcttcccga acttcttacg cccgctaagc 2172
   ggcgccttgt gcgccaacgg atacccatac caaaaccacc aacttggggg gga        2225
<210> 9
<211> 84
<212> PRT
<213> Penicillium griseofulvum
<400> 9
   Met Val Ser Phe Ser Ser Leu Phe Val Ala Ala Cys Ala Ala Val Ser
    1               5                  10                  15
   Ala Leu Ala Leu Pro Ser Asp Val Glu Lys Arg Asp Ile Thr Gln Asn
               20                  25                  30
   Glu Arg Gly Thr Asn Gly Gly Tyr Phe Tyr Ser Phe Trp Thr Asn Gly
           35                  40                  45
   Gly Gly Ser Val Ser Tyr Asn Asn Gly Asn Ala Gly Gln Tyr Ser Val
       50                  55                  60
   Asn Trp Lys Asn Cys Gly Ser Phe Thr Ser Gly Lys Gly Trp Ala Thr
   65                  70                  75                  80
   Gly Ser Ala Arg
<210> 10
<211> 133
<212> PRT
<213> Penicillium griseofulvum
<400> 10
   Asn Ile Asn Phe Ser Gly Asn Phe Asn Pro Ser Gly Asn Ala Tyr Leu
    1               5                  10                  15
   Ala Val Tyr Gly Trp Thr Lys Gly Pro Leu Val Glu Tyr Tyr Ile Met
               20                  25                  30
   Glu Asn Tyr Gly Glu Tyr Asn Pro Gly Gly Ser Met Thr Phe Lys Gly
           35                  40                  45
   Thr Val Thr Ser Asp Gly Ser Val Tyr Asp Ile Tyr Lys His Thr Gln
       50                  55                  60
   Val Asn Gln Pro Ser Ile Ile Ser Asp Ser Ser Thr Phe Asp Gln Tyr
   65                  70                  75                  80
   Trp Ser Ile Arg Arg Asn Lys Arg Ser Ser Gly Thr Val Thr Thr Gly
                   85                  90                  95
   Asn His Phe Asn Ala Trp Ala Lys Leu Gly Met Gly Leu Gly Ser His
               100                 105                 110
   Asp Tyr Gln Ile Val Asn Thr Glu Gly Tyr Gln Ser Ser Gly Ser Ala
           115                 120                 125
   Thr Ile Thr Val Ser
       130
<210> 11
<211> 217
<212> PRT
<213> Penicillium griseofulvum
<400> 11
   Met Val Ser Phe Ser Ser Leu Phe Val Ala Ala Cys Ala Ala Val Ser
   1               5                   10                  15
   Ala Leu Ala Leu Pro Ser Asp Val Glu Lys Arg Asp Ile Thr Gln Asn
               20                  25                  30
   Glu Arg Gly Thr Asn Gly Gly Tyr Phe Tyr Ser Phe Trp Thr Asn Gly
           35                  40                  45
   Gly Gly Ser Val Ser Tyr Asn Asn Gly Asn Ala Gly Gln Tyr Ser Val
       50                  55                  60
   Asn Trp Lys Asn Cys Gly Ser Phe Thr Ser Gly Lys Gly Trp Ala Thr
   65                  70                  75                  80
   Gly Ser Ala Arg Asn Ile Asn Phe Ser Gly Asn Phe Asn Pro Ser Gly
                   85                  90                  95
   Asn Ala Tyr Leu Ala Val Tyr Gly Trp Thr Lys Gly Pro Leu Val Glu
               100                 105                 110
   Tyr Tyr Ile Met Glu Asn Tyr Gly Glu Tyr Asn Pro Gly Gly Ser Met
           115                 120                 125
   Thr Phe Lys Gly Thr Val Thr Ser Asp Gly Ser Val Tyr Asp Ile Tyr
       130                 135                 140
   Lys His Thr Gln Val Asn Gln Pro Ser Ile Ile Ser Asp Ser Ser Thr
   145                 150                 155                 160
   Phe Asp Gln Tyr Trp Ser Ile Arg Arg Asn Lys Arg Ser Ser Gly Thr
                   165                 170                 175
   Val Thr Thr Gly Asn His Phe Asn Ala Trp Ala Lys Leu Gly Met Gly
               180                 185                 190
   Leu Gly Ser His Asp Tyr Gln Ile Val Asn Thr Glu Gly Tyr Gln Ser
           195                 200                 205
   Ser Gly Ser Ala Thr Ile Thr Val Ser
       210                 215
<210> 12
<211> 24
<212> DNA
<213> Artificial Sequence
<220>
<223> Description of Artificial Sequence: synthetic oligonucleotide
<400> 12
   ggaattccat aatggtctct ttct                                            24
<210> 13
<211> 25
<212> DNA
<213> Artificial Sequence
<220>
<223> Description of Artificial Sequence: synthetic oligonucleotide
<400> 13
   gctctagagc cacttgtgac atgct                                           25